# EUROPEAN PATENT SPECIFICATION

(11) **EP 1 786 358 B1**
(45) Date of publication and mention of the grant of the patent: **10.04.2013**
(21) Application number: 05754356.3
(22) Date of filing: 24.05.2005
(51) Int. Cl.: A61F 2/90

(54) **FLAP-COVER ANEURYSM STENT**
ANEURYSMASTENT MIT KLAPPENABDECKUNG
ENDOPROTHESE POUR ANEVRISME AVEC COUVERCLE RABATTABLE

(30) Priority: 09.08.2004 US 914559
(43) Date of publication of application: 23.05.2007
(73) Proprietor: Stryker Corporation, Kalamazoo, MI 49002 (US); Stryker NV Operations Limited, Dublin 2 (IE)
(72) Inventor: MUSBACH, Frank, Pleasanton, California 94588 (US)
(74) Representative: Schildberg, Peter
(86) International application number: PCT/US2005/018411
(87) International publication number: WO 2006/022948

(56) References cited:
- EP-A- 1 527 753
- WO-A-00/47134
- US-A- 5 556 413
- US-A- 5 911 733
- US-A- 5 925 063
- US-A- 6 022 371
- US-B1- 6 280 467
- US-B1- 6 676 696

## Description

### BACKGROUND OF THE INVENTION

Implantable medical devices, such as a stents, grafts, stent-grafts and the like, and delivery assemblies for implantable medical devices are utilized in a number of medical procedures and situations, and as such their structure and function are generally known in the art.

An aneurysm is generally a localized blood-filled dilation of a vessel. One method of treating an aneurysm is to place a porous stent in the vessel at the aneurysm site. A porous stent can close an aneurysm over a period of time, such as a week.

Another method of treating an aneurysm includes the use of Guglielmi electrolytically detachable coils, for example as described in US5947962. The dilation may be packed with the detachable coil, thereby obstructing blood flow such that the blood clots within the dilation, thereby forming an occlusion.

There remains a need for a device that is relatively easy to position at an aneurysm site that is capable of causing rapid stasis of blood flow.

US 6 022 371 discloses a stent which may be mechanically locked in an expanded diameter. Each cell of the stent is bisected by an arm having one end that is integrally formed with the stent and a free end.

US 5 925 063 discloses an apparatus comprising a coiled sheet having a plurality of flaps mounted on its interior surface that project radially inward into a lumen formed by the interior surface of the apparatus. The flaps form a filter e.g. for capturing material during an endarterectomy.

US 5 556 413 discloses a stent of a cylindrical body comprising a plurality of helically shaped elements capable of expansion having end assemblies capable of locking in an expanded state.

WO 00/47134 discloses a stent having a first and a second expandable portion, the latter being independently movable. A stent of this type may be used at a bifurcation of an artery, wherein the first expandable portion fixes the stent in place at a target vascular lumen and the second expandable portion is to seal an aneurismal opening. One embodiment of said second portion shows two flap structures on the second expandable portion.

US 5 911 733 A discloses a stent having a plurality of anchorage elements which are aligned around the stent in a fish scale structure for preventing migration of the stent.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

In some embodiments, a stent may comprise a framework and plurality of flaps. Flaps which are adjacent one another about the circumference of the stent desirably partially overlap one another. Flaps may include, for example, a first flap, a second flap and a third flap. A portion of the first flap may overlap a portion of the second flap. A portion of the second flap may overlap a portion of the third flap. In some embodiments, the first and third flaps do not overlap one another. The stent may have an unexpanded state and an expanded state.

In some embodiments, the flaps may be made from Nitinol and may have a thickness of 127 µm (0.0005 inches) or less. In some embodiments, the flaps may be made from one or more polymers.

In some embodiments, at least two flaps may have different lengths as measured in a longitudinal direction of the stent. In some embodiments, at least two flaps may have different shapes.

In some embodiments, the plurality of flaps may form a wall region of predetermined shape. The predetermined shape may be circular, and a plurality of radiopaque markers may be positioned about the periphery of the wall region.

In some embodiments, the stent may comprise a first closed serpentine band extending at a first end of the stent and a second closed serpentine band at a second end of the stent. One or more interconnecting elements may extend between the first and second serpentine bands. The stent may have a treatment side wherein the plurality of flaps are located, and a non-treatment side disposed opposite the treatment side. The non-treatment side may have one or more openings therethrough.

The stent comprises a plurality of overlapping flaps and the stent may be constructed and arranged such that the extent of the overlap decreases upon expansion of the stent. The flaps form a patch of a predetermined shape, which may be substantially circular when the stent is expanded.

The stent comprises a plurality of overlapping flaps, the overlapping flaps forming a patch of a predetermined shape for blocking fluid flow to a aneurysm.

In some embodiments, a stent may comprise a first serpentine band and a second serpentine band. A plurality of interconnecting elements may connect the first serpentine band to the second serpentine band. A plurality of flap struts may connect the first serpentine band to the second serpentine band. Each flap strut may support a flap. Adjacent flaps may overlap one another to form a wall region of predetermined shape. The stent may be self-expanding. In some embodiments, an interconnecting element may connect the first serpentine band to a flap or to a flap strut. In some embodiments, the region may extend a greater distance in the stent radial direction than a radius of the first serpentine band or the second serpentine band.

These and other embodiments which characterize the invention are pointed out with particularity in the claims annexed hereto and forming a part hereof. However, for a better understanding of the invention, its advantages and objectives obtained by its use, reference should be made to the drawings which form a further part hereof and the accompanying descriptive matter, in which there are illustrated and described various embodiments of the invention.

The stent according to the invention may be used in a method of treating an aneurysm. In some embodiments, a method of treating an aneurysm may comprise providing a stent comprising a plurality of overlapping flaps, the overlapping flaps forming a patch of a predetermined shape; delivering the stent to the aneurysm site; and orienting the stent such that the flaps cover the aneurysm. Desirably, the flaps block fluid flow into the aneurysm. In some embodiments, the stent may be self-expanding and the method further comprises removing a constraining sheath and allowing the stent to self-expand. In some embodiments, the stent may be balloon expandable and the method further comprises inflating a balloon to expand the stent. In some embodiments, the method further comprises positioning the stent using radiopaque markers. In some embodiments, the stent may include a plurality of radiopaque markers about the periphery of the overlapping flaps, and the method may further comprise positioning the flaps to block the aneurysm using the plurality of radiopaque markers about the periphery of the overlapping flaps.

### BRIEF DESCRIPTION OF THE DRAWINGS

A detailed description of the invention is hereafter described with specific reference being made to the drawings.
Figure 1 shows a pattern for an embodiment of an inventive stent.
Figure 2 shows a non-treatment side of an embodiment of an inventive stent.
Figure 3 shows another embodiment of an inventive stent.
Figure 4 shows an embodiment of an inventive stent deployed within a bodily vessel to block an aneurysm.
Figure 5 shows an embodiment of an inventive stent having multiple flaps on a flap strut.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

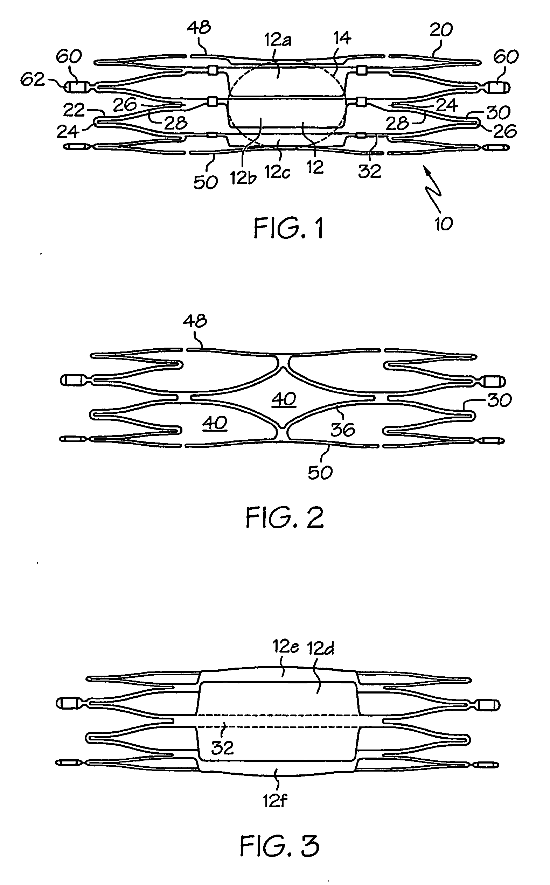

Figure 1 shows a pattern for an embodiment of a stent 10 which may comprise an expandable framework 20 and a plurality of flaps 12. The stent 10 may be positioned within a bodily vessel at an aneurysm site such that the flaps 12 are arranged to block blood flow into the aneurysm.

A stent 10 may have a treatment side and a non-treatment side. The treatment side may be defined generally by the existence of flaps 12. The treatment side may have a first edge 48 and a second edge 50 defining a divide between the treatment side and the non-treatment side. The treatment side of the stent 10 may extend about any suitable portion of the circumference of the stent 10. For example, a treatment side may extend about 90°, 180°, 270°, or any other suitable portion of the circumference. Thus, the first edge 48 and second edge 50 may be separated by 90°, 180°, 270°, or any other suitable measurement about the circumference.

Any number of the flaps 12 may collectively comprise a treatment area or patch 14. A treatment patch 14 may comprise a wall region which may have a predetermined shape. A treatment patch 14 may have any suitable shape, such as substantially circular, square, oval or other shape suitable for blocking fluid flow to an aneurysm.

Flaps 12 that are adjacent to one another desirably contact and overlap one another. For example, in some embodiments, a stent 10 may include a first flap 12a, a second flap 12b and a third flap 12c. The flaps 12 may be arranged such that a portion of the first flap 12a overlaps a portion of the second flap 12b, and a portion of the second flap 12b overlaps a portion of the third flap 12c. When one flap overlaps another flap, a theoretical line in a radial direction of the stent 10 intersects both flaps. In some embodiments, first, second and third flaps may further be arranged such that the first flap 12a does not overlap any portion of the third flap 12c.

Individual flaps 12 may be of any suitable shape and may generally have a length component and a width component The length component may be measured in a direction parallel to the central longitudinal axis of the stent 10. The width component may be measured in a direction orthogonal to the length component. When a flap 12 includes curvature, the width component may comprise a portion of the circumference of the stent 10. Various flaps 12 may be of different sizes and/or shapes than other flaps 12 within the same stent 10. A given flap 12 may have a length component that is greater than or less than the length component of another flap 12. A given flap 12 may have a width component that is greater than or less than the width component of another flap 12. Moreover, the width may vary over the length of a flap 12 and/or the length may vary over the width of a flap 12.

Flaps 12 may be formed from any suitable material, such as stainless steel, cobalt chrome alloys such as elgiloy, tantalum or other plastically deformable metals, shape-memory metals such as nickel-titanium alloys generically known as Nitinol, platinum/tungsten alloys and titanium alloys, polymeric materials such as shape-memory polymers, polyamides, polyethylenes and co-polymers, and the like.

Flaps 12 may have any suitable thickness measured in a radial direction of the stent 10. Desirably, the flaps 12 may be thin and may have a thickness ranging from 0.001 inches to 0.0004 inches (approximately 0.025 mm to 0.01 mm). In some embodiments, the flaps 12 may have a thickness ranging from 0.0025 inches to 0.0004 inches or less (approximately 0.064 mm to 0.01 mm or less). In some embodiments, the flaps 12 may have a thickness ranging from 0.0035 inches to 0.0004 inches or less (approximately 0.089 mm to 0.01 mm or less). In some embodiments, the thickness of a flap 12 may range from 0.0005 inches or less (approximately 0.013 mm or less) up to a thickness that is equal to or greater than the thickness of expandable framework 20 members.

The thickness of a flap 12 may be adjusted as required by the material used to form the flaps 12. For example, in some embodiments, flaps 12 may have a thickness of 0.005 inches or less (approximately 0.13 mm or less). Such an embodiment may optionally include flaps 12 that are formed from a polymeric material. The thickness of a flap 12 may also vary along the length and/or width of the flap 12.

Flaps 12 may be connected to the framework by any suitable method, such as by an adhesive, swaging, welding or the like. Further, polymers may be used to encapsulate portions of the flaps 12 and/or portions of the framework 20, and may be used to connect the flaps 12 to the framework 20.

In some embodiments, the flaps 12 may be made from the same material as the framework 20. In some embodiments, the flaps 12 may be formed integrally with the framework 20.

The framework 20 may be made from any suitable material, such as polymeric materials, metals, ceramics and composites. The framework 20 may be expandable from an unexpanded state to an expanded state. The stent 10 may have a nominal diameter in an expanded state. In some embodiments, the framework 20 may be made from a shape-memory material such as Nitinol, may be self-expanding, and may self-expand to the nominal diameter.

The framework 20 may comprise a first closed serpentine band 22 located at a first end of the stent 10 and a second closed serpentine band 30 located at the other end of the stent 10. Each closed serpentine band 22, 30 may be substantially cylindrically shaped and may extend about the periphery of the stent 10. Each closed serpentine band 22, 30 may comprise alternating peaks 24 and valleys 26 connected by struts 28. The closed circumferential bands 22, 30 may have the same dimensions or may have dimensions and shapes that differ. For example, a first band 22 may extend a shorter or longer distance along the length of the stent 10 than a second band 30. A first band 22 may have a larger or smaller diameter than a second band 30.

In some embodiments, the serpentine bands 22, 30 may have a nominal diameter that is less than the nominal diameter of the portion of the stent 10 having the treatment patch 14. Thus, the stent 10 may be constructed and arranged such that the treatment patch 14 extends outwardly a greater distance from the central longitudinal axis than other portions of the stent 10. The treatment patch 14 may optionally abut a vessel wall with greater pressure than other portions of the stent 10, such as framework 20 elements.

On the stent 10 treatment side, flap struts 32 may extend between framework elements located on either end of the stent 10, such as between the first serpentine band 22 and the second serpentine band 30. Each flap strut 32 may support a flap 12. In some embodiments, a flap strut 32 may extend from a valley 26 of the first serpentine band 22 to a peak 24 of the second serpentine band 30.

Figure 2 shows a pattern for an embodiment of a non-treatment side of a stent 10. Interconnecting elements 36 may extend between framework elements located on either end of the stent 10, such as between the first serpentine band 22 and the second serpentine band 30. Interconnecting elements 36 may connect to the treatment side of the stent 10. For example, interconnecting elements 36 may connect to a flap strut 32 and/or to a flap 12. Desirably, an interconnecting element may connect near the midpoint of the span of a flap 12 or flap strut 32 along its length. In some embodiments, an interconnecting element 36 may extend from a valley 26 of the first serpentine band 22 to a peak 24 of the second serpentine band 30, and may also be connected to a flap 12 or flap strut 32. In some embodiments, an interconnecting element 36 may span between a peak 24 or valley 26 and a flap strut 32 or flap 12.

Desirably, the interconnecting elements 36 may tie the first serpentine band 22 and the second serpentine band 30 together. In some embodiments, the interconnecting elements 36 may tie to a first edge 48 and/or a second edge 50 of the treatment side of the stent 10.

In some embodiments, the framework 20 may form a wall having a plurality of cells 40.

Figure 3 shows another embodiment of a treatment side of a stent 10. A first flap 12d may extend from a flap strut 32 in both directions about the circumference of the stent 10. A portion of the first flap 12d on one side of the flap strut 32 may overlap a portion of a second flap 12e. A portion of the first flap 12d on the other side of the flap strut 32 may overlap a portion of a third flap 12f.

In some embodiments, the amount of overlap between adjacent flaps 12 may change as the diameter of the stent 10 is changed. Generally, there is a greater amount of overlap in the unexpanded state than in the expanded state. The extent of the overlap may decrease upon expansion of the stent 10.

The inventive stents 10 may be manufactured using known stent manufacturing techniques. Suitable methods for manufacturing the inventive stents include laser cutting, chemical etching or stamping of a tube. The inventive stents may also be manufactured by laser cutting, chemically etching, stamping a flat sheet, rolling the sheet and, optionally, welding the sheet. Other suitable manufacturing techniques include electrode discharge machining or molding the stent with the desired design. The stent may also be manufactured by welding individual sections, for example, circumferential bands and/or the flaps, together. Any other suitable stent manufacturing process may also be used.

In some embodiments, the framework 20 may be manufactured and the flaps 12 may be attached to the framework 20 using the methods previously described

herein. The flaps 12 are formed integrally with the The flaps 12 are formed integrally with the framework 20. For example, in a preferred embodiment, a stent 10 including the flaps 12 and framework 20 elements may be laser cut from a Nitinol tube. The diameter of the tube may be larger than the nominal expanded diameter of the stent 10. The thickness of the flaps 12 may be reduced if desired, such as to a thickness of 0.0005" or less. Any suitable method may be used to reduce the thickness of the flaps 12, such as brushing, etching, electropolishing and/or micro-machining. The stent 10 may then be reduced in diameter to an unexpanded diameter and delivered to a deployment location. The stent 10 may then be expanded to a nominal diameter. Desirably, adjacent flaps 12 overlap one another when the stent is expanded to a nominal diameter.

In some embodiments wherein at least a portion of the stent 10 is formed from a shape-memory material, the stent 10 may be arranged to self-expand to a nominal diameter. In some embodiments, the stent 10 may be arranged to self-expand to a diameter that is slightly larger than the nominal diameter, and the stent 10 may be constrained to the nominal diameter by a bodily vessel. US 5197978, discusses shape-memory materials.

Referring again to Figure 1, in some embodiments, a stent 10 may include at least one and desirably a plurality of markers 60. A marker 60 may comprise a radiopaque material, a magnetic resonance contrast agent or other suitable material to allow the stent 10, or portions thereof, to be detectable within the body by fluoroscopy, MRI or other known techniques. Markers 60 may be placed near the ends of the stent 10 to show placement of the stent 10. In some embodiments, a marker 60 maybe provided on a tab 62 or other structure designed for receiving a marker 60 located at an end of the stent 10. For example, a tab 62 may be coupled to an outer peak 24 or valley 26 of a serpentine band 22, 30.

Markers 60 may also be provided adjacent to a treatment patch 14 to show placement of the treatment patch 14. For example, markers 60 may be provided on each flap strut 32 on either side of a flap 12.

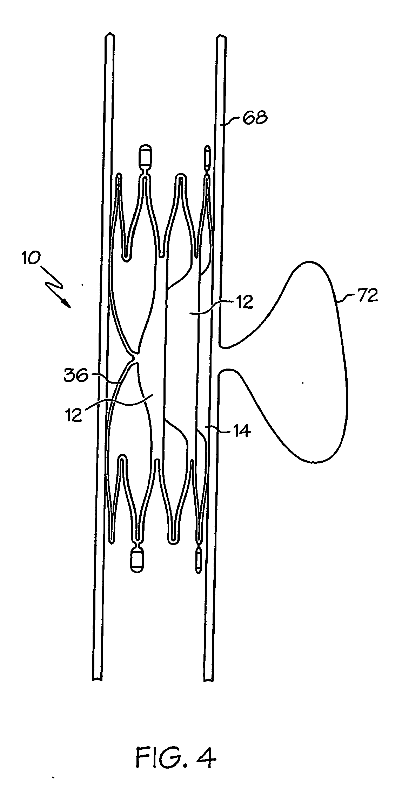

Figure 4 shows an embodiment of a stent 10 deployed within a vessel 68 with the treatment patch 14 positioned to block fluid flow to an aneurysm 72. Framework 20 elements desirably abut the vessel 68 wall while the flaps 12 desirably cover the aneurysm 72.

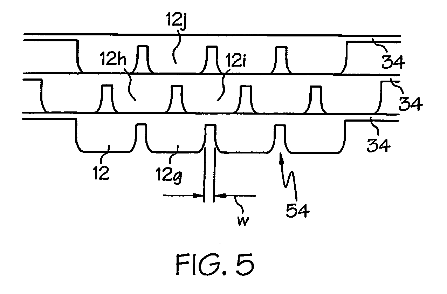

Referring to Figure 5, in some embodiments, a flap strut 32 may include multiple flaps 12 along its length. Flap struts 32 having multiple flaps 12 may provide the stent with greater flexibility along its longitudinal axis, and may improve deliverability of the stent through a tortuous anatomy.

A given flap strut 32 may include any number of flaps 12. Flaps 12 may have any suitable size and shape. Adjacent flaps 12 may have similar dimensions or differing dimensions. For example, in some embodiments, flaps 12 which are located near the midpoint of a flap strut 32 may extend farther in a stent circumferential direction than flaps 12 which are located near an end of the flap strut 32.

In some embodiments, the sides of adjacent flaps 12 may abut one another. In some embodiments, adjacent flaps 12 may be separated by a kerf or gap 54. The width w of a gap 54 at the closest point between adjacent flaps 12 may range from zero (no gap) to any suitable dimension. For example, the width w of a gap 54 may range from zero to 0.008 inches or more (0 to 0.2 mm or more). Desirably, the width w of a gap 54 may range from 0.003 inches to 0.005 inches (approximately 0.076 mm to 0.13 mm). It should be noted that the Figures are not drawn to scale.

In some embodiments, the flaps 12 of circumferentially adjacent flap struts 32 may be longitudinally aligned. For example, flaps 12g and 12j as shown in Figure 5 are longitudinally aligned. A reference circumference drawn about the stent which passes through a midpoint of flap 12g will also pass through a midpoint of flap 12j. In some embodiments, the flaps 12 ofcircumferentially adjacent flap struts 32 may be staggered or longitudinally offset. For example, flaps 12h and 12i as shown in Figure 5 are longitudinally offset from flaps 12g and 12j.

Desirably, flaps 12 may include rounded edges 44.

The stent according to the invention may be used in a method of treating an aneurysm 72. A stent 10 having flaps 12 maybe reduced to an unexpanded diameter and secured to a delivery device, such as a delivery catheter. The catheter may be advanced through the vessel 68 until the stent 10 is delivered to the aneurysm site. The stent 10 may be oriented such that the flaps 12 are positioned to cover the aneurysm 72. The stent 10 may be expanded, for example by inflating an expansion balloon, or if the stent 10 is self-expanding, by removing a constraining sheath. The catheter may then be removed, leaving the stent 10 positioned such that the flaps 12 block fluid flow into the aneurysm 72.

In some embodiments, a stent may be formed according to the following numbered paragraphs:
1. A stent comprising a framework and plurality of flaps including a first flap connected to a first flap strut, a second flap connected to a second flap strut and a third flap connected to the second flap strut; wherein the first flap partially overlaps the second flap.
2. The stent of paragraph 1 above, wherein the first flap partially overlaps the third flap.
3. The stent of paragraph 1 above, wherein the first flap is longitudinally aligned with the second flap.
4. A stent comprising an expandable framework including at least one flap strut, the flap strut including a first flap and a second flap.
5. The stent of paragraph 4 above, further comprising a second flap strut including a third flap.
6. The stent of paragraph 5 above, wherein the first flap partially overlaps the third flap.
7. The stent of paragraph 4 above, wherein a side of the first flap abuts a side of the second flap.
8. The stent of paragraph 4 above, wherein the first flap and the second flap are separated by a gap.
9. The stent of paragraph 8 above, wherein a width of the gap ranges from 0.003 inches to 0.005 inches.
10. The stent of paragraph 5 above, wherein the first flap is longitudinally aligned with the third flap.
11. The stent of paragraph 5 above, wherein the first flap is longitudinally offset from the third flap.

Any of the inventive stents disclosed above may be provided with a uniform diameter or may taper in portions or along the entire length of the stent. Also, the width and/or thickness of the various portions of the inventive stents, such as framework elements, may increase or decrease along a given portion of the stent. For example, the width and/or thickness of the circumferential bands and/or interconnecting elements may increase or decrease along portions of the stent or along the entire length of the stent.

It is also within the scope of the invention for any of the stents disclosed herein to have interconnecting elements and/or flap struts extending from regions other than peaks and valleys of the serpentine bands. For example, the interconnecting elements and/or flap struts may extend from positions between adjacent peaks and valleys, such as from positions one quarter of the way between peaks and valleys, from positions one-half of the way between peaks and valleys, from positions three quarters of the way between peaks and valleys or anywhere else along a strut.

The invention also contemplates the use of more than one material in the inventive stents. For example, framework elements may be made from different materials than the flaps. In some embodiments, different portions of the framework may be made from different materials. For example, the interconnecting elements may be made from different materials than the serpentine bands.

The inventive stents may be provided in mechanically expandable form, in self-expanding form or as a hybrid of the two. Mechanically expandable stents, in accordance with the invention, may be expanded using any suitable mechanical device including a balloon.

The inventive stents may include suitable radiopaque coatings. For example, the stents may be coated with gold or other noble metals or sputtered with tantalum or other metals. The stents may also be made directly from a radiopaque material to obviate the need for a radiopaque coating or may be made of a material having a radiopaque inner core. Other radiopaque metals which may be used include platinum, platinum-tungsten, palladium, platinum-iridium, rhodium, tantalum, or alloys or composites of these metals. Markers 60 as disclosed herein may be made from any of the above-listed materials.

The inventive stents may also be provided with various bio-compatible coatings to enhance various properties of the stent. For example, the inventive stents may be provided with lubricious coatings. The inventive stents may also be provided with drug-containing coatings which release drugs over time. The increased surface area of a stent having bent struts provides for increased drug coatability. The bent struts also provide for point contact with a crimper versus strut/strut contact Less contact with the crimper results in less disruption of the drug coating.

The inventive stents may also be provided with a sugar or more generally a carbohydrate and/or a gelatin to maintain the stent on a balloon during delivery of the stent to a desired bodily location. Other suitable compounds for treating the stent include biodegradable polymers and polymers which are dissolvable in bodily fluids. Portions of the interior and/or exterior of the stent may be coated or impregnated with the compound. Mechanical retention devices may also be used to maintain the stent on the balloon during delivery. To that end, the use of other coatings on the inventive stents is also within the scope of the invention.

The coating may comprise one or more non-genetic therapeutic agents, genetic materials and cells and combinations thereof as well as other polymeric coatings.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The inventive stents may also be provided with a graft material. A graft material may be applied to any portion of the stent, such as the framework and/or the flaps. Suitable coverings include nylon, collagen, PTFE and expanded PTFE, polyethylene terephthalate and KEVLAR®, or any of the materials disclosed in US 5,824,046 and US 5,755,770. More generally, any known graft material may be used including synthetic polymers such as polyethylene, polypropylene, polyurethane, polyglycolic acid, polyesters, polyamides, their mixtures, blends and copolymers.

The inventive stents may find use in coronary arteries, renal arteries, peripheral arteries including iliac arteries, arteries of the neck and cerebral arteries. The stents of the present invention, however, are not limited to use in the vascular system and may also be advantageously employed in other body structures, including but not limited to arteries, veins, biliary ducts, urethras, fallopian tubes, bronchial tubes, the trachea, the esophagus, the prostate and the bowels.

Suitable stent delivery devices such as those disclosed in US 6,123,712, US 6,120,522 and US 5,957,930 may be used to deliver the inventive stents to the desired bodily location. The choice of delivery device will depend on whether a self-expanding or balloon expandable stent is used. The inventive stents may be delivered in conjunction with one or more stent retaining sleeves. An example of stent retaining sleeves is disclosed in US provisional application 09/970459.

The above disclosure is intended to be illustrative and not exhaustive. This description will suggest many variations and alternatives to one of ordinary skill in this field of art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

This completes the description of the embodiments of the invention. Those skilled in the art may recognize other equivalents to the specific embodiment described herein which equivalents are intended to be encompassed by the claims attached hereto.

This PCT application claims priority from US Application No. 10/914559, filed on 9 August 2004.

## Claims

1. A stent comprising a plurality of overlapping flaps (12), **characterised in that** the overlapping flaps (12) overlap when the stent is in an expanded state, and are constructed integrally with the stent and form a patch (14) of a predetermined shape for blocking fluid flow to an aneurysm and, in the area of overlap, a theoretical line in a radial direction of the stent intersects two flaps.

2. The stent of claim 1 wherein the patch (14) is substantially circular.

3. The stent of claim 1 or 2 wherein the flaps (12) include a first flap (12a), a second flap (12b) and a third flap (12c), a portion of the first flap overlapping a portion of the second flap, a portion of the second flap overlapping a portion of the third flap

4. The stent of any of claims 1 to 3 wherein the first (12a) and third (12c) flaps do not overlap one another.

5. The stent of any of claim 1 to 4 wherein the flaps (12) have a thickness of 0.0035 inches (0.09 mm) to 0.0004 inches (0.01 mm).

6. The stent of any of claims 1 to 5 wherein the flaps (12) are made from Nitinol.

7. The stent of any of claims 1 to 6 wherein the flaps (12) are made from one or more polymers.

8. The stent of any of claims 1 to 7 wherein the plurality of flaps (12) includes at least two flaps of different lengths as measured in a longitudinal direction of the stent.

9. The stent of any of claims 1 to 8 wherein the plurality of flaps (12) includes at least two flaps of different shapes.

10. The stent of any of claims 1 to 9 wherein the plurality of flaps (12) extends over only a portion of the stent.

11. The stent of any of claim 1 to 10 wherein a plurality of radiopaque markers (60) are positioned about the periphery of the patch.

12. The stent of any of claims 1 to 11 comprising a first serpentine band (22); a second serpentine band (30); a plurality of interconnecting elements (36) connecting the first serpentine band to the second serpentine band; and a plurality of flap struts (32) connecting the first serpentine band to the second serpentine band; each flap strut having a flap extending therefrom.

13. The stent of any of claim 12, wherein a flap strut (32) includes at least two flaps.

14. The stent of claim 12 or 13 wherein the first serpentine band (22) extends at a first end of the stent and the second serpentine (30) band at a second end of the stent.

15. The stent of any of claims 1 to 14 having a treatment side wherein the plurality of flaps are located and a non-treatment side disposed opposite the treatment side, the non-treatment side having one or more openings therethrough.

16. The stent of any of claims 1 to 15, wherein the stent is constructed and arranged such that the extent of the overlap between a first flap and a second flap decreases upon expansion of the stent.

17. The stent of any of claims 1 to 16, wherein the stent is self-expanding.

18. The stent of any of claims 12 to 17, wherein the patch (14) extends a greater distance in the stent radial direction than a radius of the first serpentine band (22).

## Patentansprüche

1. Stent, umfassend mehrere überlappende Klappen (12), **dadurch gekennzeichnet, dass** die überlappenden Klappen (12), wenn sich der Stent im expandierten Zustand befindet, überlappen und dass sie als ein Bauteil mit dem Stent gebaut sind, und ein Stück (14) einer vorbestimmten Form zum Blockieren des Fluidflusses zu einem Aneurysma bilden, und dass in der Überlappungsfläche, eine theoretische Linie in radialer Richtung des Stents zwei Klappen schneidet.

2. Stent nach Anspruch 1, wobei das Stück (14) im Wesentlichen kreisförmig ist.

3. Stent nach Anspruch 1 oder 2, wobei die Klappen (12) eine erste Klappe (12a), eine zweite Klappe (12b) und eine dritte Klappe (12c) umfassen, wobei ein Abschnitt der ersten Klappe einen Abschnitt der zweiten Klappe überlappt, ein Abschnitt der zweiten Klappe einen Abschnitt der dritten Klappe überlappt.

4. Stent nach einem der Ansprüchen 1 bis 3, wobei die erste (12a) und dritte (12c) Klappe einander nicht überlappen.

5. Stent nach einem der Ansprüche 1 bis 4, wobei die Klappen (12) eine Dicke von 0,0035 Zoll (0,09 mm) bis 0,0004 Zoll (0,01 mm) aufweisen.

6. Stent nach einem der Ansprüche 1 bis 5, wobei die Klappen (12) aus Nitinol hergestellt sind.

7. Stent nach einem der Ansprüche 1 bis 6, wobei die Klappen (12) aus einem oder mehreren Polymeren hergestellt sind.

8. Stent nach einem der Ansprüche 1 bis 7, wobei die mehreren Klappen (12) mindestens zwei Klappen unterschiedlicher Länge, gemessen in einer Längsrichtung des Stents, umfassen.

9. Stent nach einem der Ansprüche 1 bis 8, wobei die mehreren Klappen (12) mindestens zwei Klappen unterschiedlicher Formen umfassen.

10. Stent nach einem der Ansprüche 1 bis 9, wobei sich die mehreren Klappen (12) nur über einen Abschnitt des Stents erstrecken.

11. Stent nach einem der Ansprüche 1 bis 10, wobei mehrere strahlenundurchlässige Marker (60) um den Umfang des Stücks positioniert sind.

12. Stent nach einem der Ansprüche 1 bis 11, umfassend ein erstes schlangenartig gewundenes Band (22); ein zweites schlangenartig gewundenes Band (30); mehrere verbindende Elemente (36), die das erste schlangenartig gewundene Band mit dem zweiten schlangenartig gewundenen Band verbinden; mehrere Klappenstreben (32), die das erste schlangenartig gewundene Band mit dem zweiten schlangenartig gewundenen Band verbinden; wobei jede Klappenstrebe eine Klappe aufweist, die sich davon erstreckt.

13. Stent nach einem von Anspruch 12, wobei eine Klappenstrebe (32) mindestens zwei Klappen umfasst.

14. Stent nach Anspruch 12 oder 13, wobei sich das erste schlangenartig gewundene Band (22) an einem ersten Ende des Stents erstreckt und das zweite schlangenartig gewundene Band (30) an einem zweiten Ende des Stents.

15. Stent nach einem der Ansprüche 1 bis 14, der eine Behandlungsseite aufweist, an der sich die mehreren Klappen befinden, und eine Nicht-Behandlungsseite, die gegenüberliegend der Behandlungsseite angeordnet ist, wobei die Nicht-Behandlungsseite eine oder mehrere Öffnungen darin aufweist.

16. Stent nach einem der Ansprüche 1 bis 15, wobei der Stent so gebaut und angeordnet ist, dass das Ausmaß der Überlappung zwischen einer ersten Klappe und einer zweiten Klappe bei Ausweitung des Stents abnimmt.

17. Stent nach einem der Ansprüche 1 bis 6, wobei der Stent selbstexpandierend ist.

18. Stent nach einem der Ansprüche 12 bis 17, wobei sich das Stück (14) über einen größeren Abstand in der radialen Richtung des Stents erstreckt, als ein Radius des ersten schlangenartig gewundenen Bandes (22).

## Revendications

1. Endoprothèse comprenant une pluralité de rabats à recouvrement (12), **caractérisée en ce que** les rabats à recouvrement (12) se chevauchent lorsque l'endoprothèse se trouve dans un état expansé, et sont construits d'un seul tenant avec l'endoprothèse et forment une pièce (14) d'une forme prédéterminée destinée à bloquer l'écoulement de fluide au niveau d'un anévrisme et, dans la zone de recouvrement, une ligne théorique dans une direction radiale de l'endoprothèse croise deux rabats.

2. Endoprothèse selon la revendication 1, dans laquelle la pièce (14) est sensiblement circulaire.

3. Endoprothèse selon les revendications 1 ou 2, dans laquelle les rabats (12) comprennent un premier rabat (12a), un deuxième rabat (12b) et un troisième rabat (12c), une partie du premier rabat chevauchant une partie du deuxième rabat, une partie du deuxième rabat chevauchant une partie du troisième rabat.

4. Endoprothèse selon l'une quelconque des revendications 1 à 3, dans laquelle les premier (12a) et troisième (12c) rabats ne se chevauchent pas mutuellement.

5. Endoprothèse selon l'une quelconque des revendications 1 à 4, dans laquelle les rabats (12) présentent une épaisseur de 0,0035 pouces (0,09 mm) à 0,0004 pouces (0,01 mm).

6. Endoprothèse selon l'une quelconque des revendications 1 à 5, dans laquelle les rabats (12) sont en nitinol.

7. Endoprothèse selon l'une quelconque des revendications 1 à 6, dans laquelle les rabats (12) sont formés d'un ou de plusieurs polymères.

8. Endoprothèse selon l'une quelconque des revendications 1 à 7, dans laquelle la pluralité des rabats (12) comprend au moins deux rabats de différentes longueurs, telle que mesurée dans une direction longitudinale de l'endoprothèse.

9. Endoprothèse selon l'une quelconque des revendications 1 à 8, dans laquelle la pluralité des rabats (12) comprend au moins deux rabats de différentes formes.

10. Endoprothèse selon l'une quelconque des revendications 1 à 9, dans laquelle la pluralité de rabats (12) ne s'étend que sur une partie de l'endoprothèse.

11. Endoprothèse selon l'une quelconque des revendications 1 à 10, dans laquelle une pluralité de marqueurs radioopaques (60) est positionnée sur la périphérie de la pièce.

12. Endoprothèse selon l'une quelconque des revendications 1 à 11, comprenant une première bande en serpentin (22) ; une seconde bande en serpentin (30) ; une pluralité d'éléments d'interconnexion (36) reliant la première bande en serpentin à la seconde bande en serpentin ; et une pluralité d'entretoises de rabat (32) reliant la première bande en serpentin à la seconde bande en serpentin ; chaque entretoise de rabat ayant un rabat s'étendant depuis celle-ci.

13. Endoprothèse selon une la revendication 12, dans laquelle une entretoise de rabat (32) comprend au moins deux rabats.

14. Endoprothèse selon les revendications 12 ou 13, dans laquelle la première bande en serpentin (22) s'étend sur une première extrémité de l'endoprothèse et la seconde bande en serpentin (30) sur une seconde extrémité de l'endoprothèse.

15. Endoprothèse selon l'une quelconque des revendications 1 à 14, ayant une face de traitement dans laquelle est localisée la pluralité de rabats et une face de non-traitement étant située à l'opposé de la face de traitement, la face de non-traitement étant traversée par une ou plusieurs ouvertures.

16. Endoprothèse selon l'une quelconque des revendications 1 à 15, dans laquelle l'endoprothèse est construite et disposée de telle sorte que l'étendue du recouvrement entre un premier rabat et un second rabat diminue avec l'expansion de l'endoprothèse.

17. Endoprothèse selon l'une quelconque des revendications 1 à 16, dans laquelle l'endoprothèse est auto-expansible.

18. Endoprothèse selon l'une quelconque des revendications 12 à 17, dans laquelle la pièce (14) s'étend sur une distance supérieure dans la direction radiale d'endoprothèse à un rayon de la première bande en serpentin (22).
